# EUROPEAN PATENT SPECIFICATION

(11) **EP 1 048 726 B1**
(45) Date of publication and mention of the grant of the patent: **26.07.2006**
(21) Application number: 00104677.0
(22) Date of filing: 03.03.2000
(51) Int. Cl.: C12N 15/12, C12N 15/67, C07K 14/755

(54) **Modified factor VIII cDNA**
Modifizierte Faktor VIII cDNA
ADN modifier du factor VIII

(30) Priority: 17.03.1999 EP 99104050
(43) Date of publication of application: 02.11.2000
(73) Proprietor: Négrier, Claude, 69437 Lyon Cédex 03 (FR)
(72) Inventor: Negriér, Claude, Dr., 69540 Irigny (FR); Plantier, Jean Luc, 69520 Grigny (FR)
(74) Representative: Lepeudry-Gautherat, Thérèse

(56) References cited:
- EP-A- 1 038 959
- WO-A-92/16557
- WO-A-94/29471
- FR-A- 2 763 959
- LIND ET AL: "Novel forms of B-domain-deleted recombinant factor VIII molecules. Construction and biochemical characterization" EUROPEAN JOURNAL OF BIOCHEMISTRY,DE,BERLIN, vol. 232, no. 1, August 1995 (1995-08), pages 19-27, XP002111900 ISSN: 0014-2956
- KURACHI S ET AL: "ROLE OF INTRON I IN EXPRESSION OF THE HUMAN FACTOR IX GENE" JOURNAL OF BIOLOGICAL CHEMISTRY,US,AMERICAN SOCIETY OF BIOLOGICAL CHEMISTS, BALTIMORE, MD, vol. 270, no. 10, 10 March 1995 (1995-03-10), pages 5276-5281, XP002055415 ISSN: 0021-9258
- PLANTIER J -L ET AL: "A combination of truncated factor IX intron I highly improves FVIII production." BLOOD, vol. 94, no. 10 SUPPL. 1 PART 1, 15 November 1999 (1999-11-15), page 454a XP002148565 Forty-first Annual Meeting of the American Society of Hematology;New Orleans, Louisiana, USA; December 3-7, 1999 ISSN: 0006-4971

## Description

This invention is directed to a modified Factor VIII cDNA and its use for the improvement of the Factor VIII production.

Factor VIII (FVIII) is a plasma coagulation cofactor implicated in the activation of Factor X (FX). A decrease in the presence or activity of Factor VIII in blood stream leads to hemophilia A. The level of the decrease in Factor VIII activity is directly proportional to the severity of the disease (Foster and T.S., 1989; Kaufman, 1992; Vlot et al., 1998). The current treatment of hemophilia A consists of the replacement of the missing protein by plasma-derived or recombinant FVIII. Recombinant FVIII is produced in CHO or BHK cells after selection of the best producing clones and amplification of the FVIII cDNA copy number.

Several studies have stressed the low FVIII production level in different cellular systems: Biosynthesis of FVIII was shown to be regulated in at least three different levels. First, among the FVIII cDNA sequence two nucleotides stretches, localized in the A2 coding domain, were demonstrated to act as transcriptional silencers (Fallaux et al, 1996; Hoeben et al., 1995; Koeberl et al., 1995; Lynch et al., 1993). Second, FVIII protein synthesis is tightly regulated by several reticulum endoplasmic chaperones (BiP; Calreticulin; Calnexin; ERGIC-53). Many of these interactions retain FVIII in the cell and direct it through the cellular degradation machinery (Dorner et al., 1987; Nichols et al., 1998; Pipe et al., 1998). Third, once secreted FVIII is sensitive to protease degradation and needs to be protected by von Willebrand Factor (vWF) (Kaufman et al., 1989).

Document WO 92 16557 describes the expression of biologically active B-domain-depleted recombinant human FVIII in Chinese hamster ovary cells after vector-mediated gene transduction.
Document WO 94 29471 describes an adenoviral vector expressing FVIII or FIX and wherein said vector further includes at least one genomic element. The vector can be used for gene therapy for delivering clotting factors such as FVIII or FIX for the treatment of hemophilia. Nevertheless, the use of this invention is especially difficult with FVIII.
Document Lind et al., 1995 describes the production of recombinant molecules similar to the smallest active plasma-derived FVIII molecule, a complex of an 80 kDa and 90 kDa polypeptide chain lacking the B domain, produced using various FVIII cDNA constructs in order to obtain primary translation products whih were efficiently processed into the 80+90 kDa complex. Nevertheless, this FVIII doesn't present particular advantages on FVIII expression yields.
The document Kurachi et al., 1995 describes the positive effect of intron I respectively largely truncated intron I on the factor IX expression. Nevertheless, the insertion of such an intron into FIX does not indicate any usability in FVIII.
The Inventors have already worked on the FVIII expression, as illustrated in the document EP 1 038 959.

It is therefore a problem to develop improved processes which result in higher yields of FVIII. The present invention offers a solution to this problem by a modified FVIII cDNA.
According to this invention a modified FVIII cDNA is made available in wich the B-domain of the wild-type FVIII cDNA has been deleted and a truncated FIX intron 1 has been inserted in one or more locations of the FVIII cDNA.
In addition the B-domain of the wild-type FVIII cDNA has been replaced by four arginines.
These FVIII constructs were prepared as follows:

### 1. FVIII cloning

A PCR cloning strategy was designed, based on the synthesis of four PCR fragments, spanning the FVIII cDNA and excepting the B-domain. Based on published data, it was of interest to replace the B-domain by four arginines. Using the MoMuLV reverse transcriptase (Promega, Charbonnières, France) a reverse transcription was done on human cell RNA isolated from a liver biopsy with the written informed consent of the patient. Four PCR fragments were generated using the Expand System (Boehringer-Mannheim, Germany), as described in Table 1.

| Fragment | Oligonucleotide sens antisens | SEQ ID No. | Size in bp | 5' cloning site | 3' cloning site |
|---|---|---|---|---|---|
| 1 | FVIII ATG | No.1 | 1704 | Not I | Bgl II |
| | 3'Bgl II | No.2 | | | |
| 2 | 5'Bgl II | No.3 | 624 | Bgl II | Sal I |
| | 4R AS | No.4 | | | |
| 3 | 4R S | No.5 | 1093 | Sal I | Bgl I |
| | 3'Bg I | No.6 | | | |
| 4 | 5'Bgl I | No.7 | 1026 | Bgl I | Xho I |
| | FVIII stop | No.8 | | | |

### Table I: Summary of FVIII PCR fragments

All the oligonucleotide sequences used for cloning are shown in Annex 1.

An additional improvement was performed by optimisation of the ATG environment following the rules of Kozak (Kozak, 1997). For this purpose the oligonucleotide FVIII ATG (SEQ ID No 1) was used.

### Comparison of the wild-type FVIII ATG and the Kozak modified FVIII ATG sequences

WT sequence: TAA GTC ATG CAA ATA
Kozak modified: ACA CCC ATG GAA ATA

The modified amino-acids are represented in bold.

Four arginines replace according to the invention the B-domain of the FVIII protein. They are introduced by the oligonucleotides used for the cloning of the two fragments surrounding the B-domain (see Fragments 2 and 3 of Table I), namely the oligonucleotides 4R AS (SEQ.ID.No.4) and 4R S (SEQ.ID.No.5). The Sal I site was generated by the coding sequence of the arginines as follows:

SAL I SITE

4R S :5'-A AGA CGT CGA CGA GAA ATA ACT CGT ACT ACT CTT
4R AS TTG TTA CGG TAA CTT GGT TCT TCT GCA GCT GCT CTT

### CORRESPONDING PEPTIDIC SEQUENCE:

Pro Arg Arg Arg Arg Glu Ile Thr Arg Thr Thr Leu
In the wild-type FVIII the peptidic sequence is:
Pro-Arg-Domain B-Arg-Glu

This indicates that a Sal I restriction site was inserted through the middle of the fourth arginine coding sequence without any sequence alteration. All PCR fragments were cloned in a pCR2 vector using the T/A cloning kit (InVitrogen, the Netherlands). Two clones of each fragment were entirely sequenced. Some mutations were found in a ratio of 1 for 800 bases. One mutation was silent but three others modified the coding sequence and the three cDNA pieces bearing the mutations were successively exchanged.

A subsequent sub-cloning strategy leads to the production of two fragments (each being the sum of two PCR products): a 5' 2.3kb (FVIII ATG) and a 3' 2.1kb (FVIII Stop). The 5' 2.3kb (FVIII ATG) and a 3' 2.1kb (FVIII Stop) were constructed and inserted in the expression vector pCDNA3 (InVitrogen, The Netherlands) opened by Not I and Xho I and treated with alkaline phosphatase. The cloning cassette of pCDNA3 presents a similar restriction sequence as pCR2 and the vector possesses its own resistance gene (neomycin). A B-domain deleted FVIII coding cDNA (hereafter refered to Factor VIII cDNA) was directly obtained in the expression vector. The final FVIII cDNA integrity was checked by extensive restriction analysis.

Factor VIII cDNA was subcloned in the Bluescript pKS II+vector opened by Not I and Xho I. The use of this vector was more convenient for the introduction of subsequent modifications in Factor VIII cDNA (e.g. introns addition; see hereafter).

### 2. Truncated FIX intron 1 insertion

According to the invention the FVIII cDNA was further modified by the insertion of a Factor IX truncated intron 1 (FIX TI1=SEQ.ID.No.9). The FIXTI1 was inserted in different locations of the FVIII cDNA as follows:
- in the FVIII intron 1 location, to use a similar strategy as for FIX (Kurachi et al., 1995)
- in the FVIII intron 12 and 13 locations, because a transcriptional silencer sequence was described in this region.
- in the FVIII introns 1+12 and 1+13 locations. Since FVIII cDNA is much larger than FIX (4.4 kb vs 1.4 kb), we hypothesized that it might be of interest to introduce supposedly stabilizing sequences at the locations normally occupied by introns 12 and 13 in addition to intron 1. Since the location of introns 12 and 13 are grossly in the middle of the FVIII sequence it is possible that they may act synergistically with intron 1.

The FIXT1I-Sequence (=SEQ-ID No. 9)used according to the invention in different locations of the FVIII cDNA starts after the coding sequence by the splice donor sequence and ends by the splice acceptor sequence of the truncated intron 1. The upper case letters start after and stop before the Nsi I and Mlu I restriction sites, respectively. For details see Annex 2.

For cloning this fragment in an exogenous sequence, two new FVIII junction fragments were generated, one upstream the FIX TI1 with the addition of a Nsi I site and one downstream with the addition of a Mlu I site. The three fragments were subsequently linked together using these sites.

A similar strategy was used for inserting the three FIXTI1 in different locations. In each case three PCR fragments (A, B, C) were generated with the Expand System using Factor VIII cDNA as template for segments A and C, and Factor IX intron 1 for the B fragment. The A fragment extremities comprise Factor VIII sequence on the 5' end, and on the 3' end a fusion between the FVIII 3' splicing sequence and the Factor IX first intron 5' splicing sequence. A Nsi I restriction site was added between these two sequences. The B fragment possesses at the 5' extremity a complementary sequence to the previous fragment, the truncated Factor IX intron 1, and at the 3' end an inserted Mlul restriction site. The C fragment was made of the complementary sequence of the 3' extremity from fragment B followed by the Factor IX first intron 3' splicing sequence and by the Factor VIII cDNA downstream coding sequence (see Figure I).

The B fragment was the same for all constructs whereas A and C were all different, corresponding to the 5' and 3' FVIII sequences of the intron insertion sites.

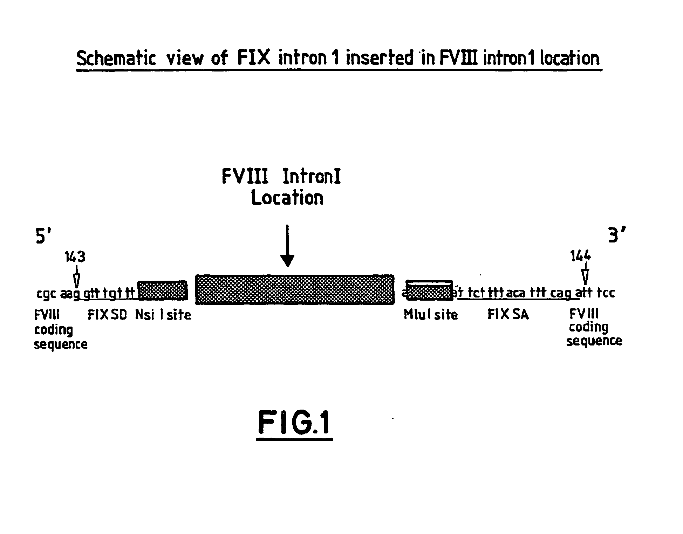

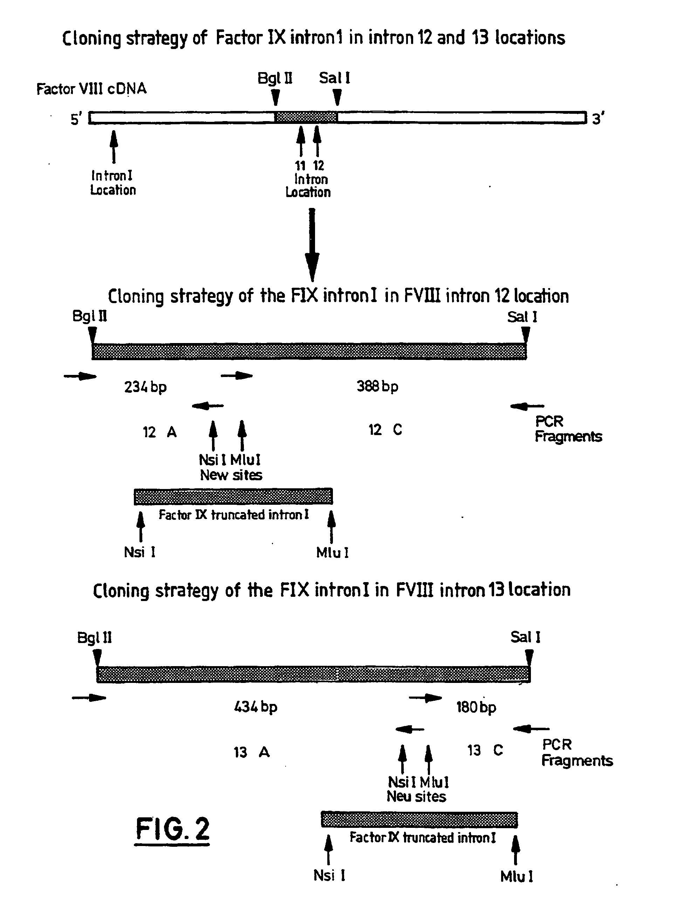

The insertion strategies for intron 12 and 13 positions are indicated in Figure 2.

Each PCR fragment was generated and first cloned in pCR2 vector using T/A cloning kit (InVitrogen, The Netherlands). They were linked together in two successive steps (pCR2-B+C/MluI+XhoI; pCR2-A+BC/NsiI+XbaI). All ABC (1, 12 and 13) fragments were sequenced and shown to be free of mutations.

| Fragment Name | Size in bp |
|---|---|
| I1-A | 204 |
| TFIXI1 (B) | 281 |
| I1-C | 464 |
| I12-A | 234 |
| I12-C | 388 |
| I13-A | 434 |
| I13-C | 180 |

### Table II: Summary of the PCR fragments required for introducing TFIXI1.

ABC1 was cloned in pKS II+ FVIII after Spe I-Spe I digestion. After checking the orientation, the resulting FVIII I1 cDNA was subcloned in pCDNA3 vector using a Not I-Xho I digestion. ABC12 and ABC13 were digested by Bgl II and Sal I and ligated in pKSII-FVIII digested by the same enzymes. Resulting FVIII I12 and FVIII I13 were subcloned in pCDNA3 using a Not I-Xho I restriction.

To generate a construct containing two introns, pKS FVIII I1 and the fragments ABC 12 or ABC 13 were digested by BgI II and Sal I and ligated together. pKSII FVIII I1+12 and FVIII I1+13 were obtained and FVIII cDNAs containing the introns were subcloned in pCDNA3 by the same strategy as previously described.

### 3. Generation of stably expressing FVIII cell lines

All pCDNA3-FVIII constructs were transfected in CHO cells by electroporation. Briefly, 7x10⁶ washed CHO cells were electroporated in the presence of 10 g of Pvu I linearized construct. The cells were selected 15 hours after electroporation in IMDM (=Iscove's Modified Dulbecco's Medium) supplemented by antibiotics and 10% fetal calf serum containing 0.6 mg/ml G418. For each construct (FVIII; I1, I12; I13; I1+12; I1+13), two pools of clones containing more than 50 individual colonies were grown and frozen. A set of 25 individual colonies were picked up, grown and assessed for Factor VIII expression using an ELISA method (Asserachrom VIIIC.Ag, Diagnostica STAGO, Asnières, France). For all clones, the transfection efficiency was around 40-60% as determined by the number of expressing clones. The five best FVIII producer clones were kept and frozen. The FVIII antigen detected varied dramatically from one construct to another.

The activity of the recombinant Factor VIII was measured using the classical one stage clotting assay for each pool and five selected clones. A coagulant activity was found in each supernatant of Factor VIII expressing cells indicating that the construct was coding for a functional protein. The activity was correlated to the amount of protein detected by ELISA. Therefore, it was demonstrated that each construct allowed the production of a functional procoagulant FVIII.

### 4. Evaluation of the construct efficiency

### 4.1 Quantitative analysis in CHO cells

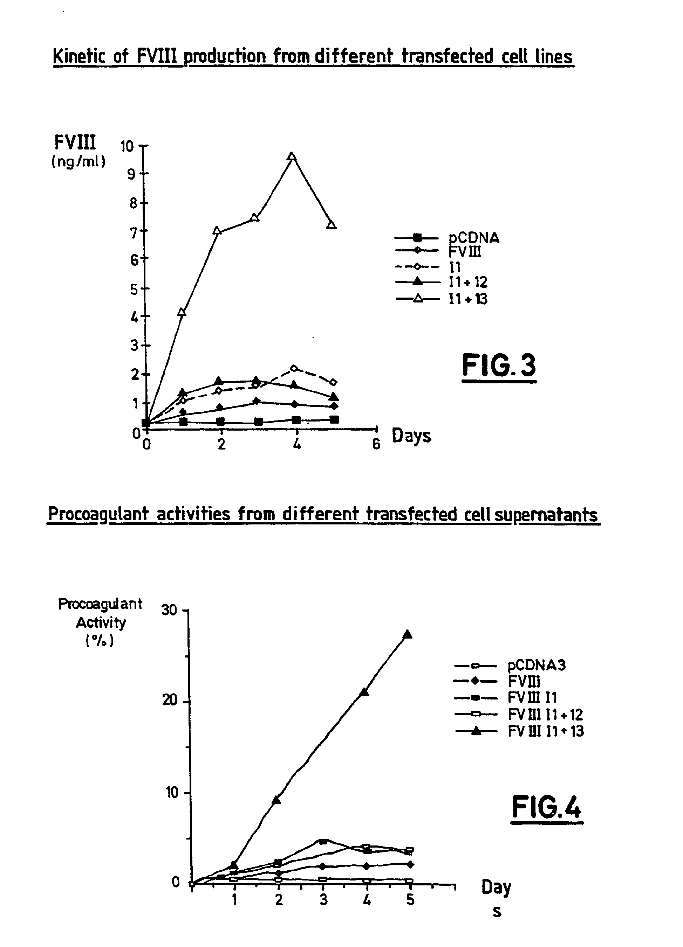

The kinetic of FVIII production was analyzed for each pool of transfected CHO cells. For each pool, independent experiments were made by two different experimentators. At day 0,4.10⁵ cells were seeded in a 6-well plate in 2 ml of fresh medium containing 0.6 mg/ml G418. From day 1 to day 4, the culture supernatant was collected, centrifuged and assayed for FVIII antigen and procoagulant activity. Each day, after medium removal, cells were trypsinized and counted in Trypan Blue. FVIII accumulation is shown in Figure 3. Three constructs allowed a better production than the non-modified FVIII cDNA, i.e. FVIII I1, FVIII I1+12 and particularly FVIII I1+13. FVIII I1 and FVIII I1+12 led to a 2 to 3 times higher production and FVIII I1+13 to 8 to 9 times more. Procoagulant activities were measured from the same supernatants and are presented in Figure 4. They were directly correlated to the amount of antigen detected.

### 4.2 Quantitative analysis in HepG2 cells

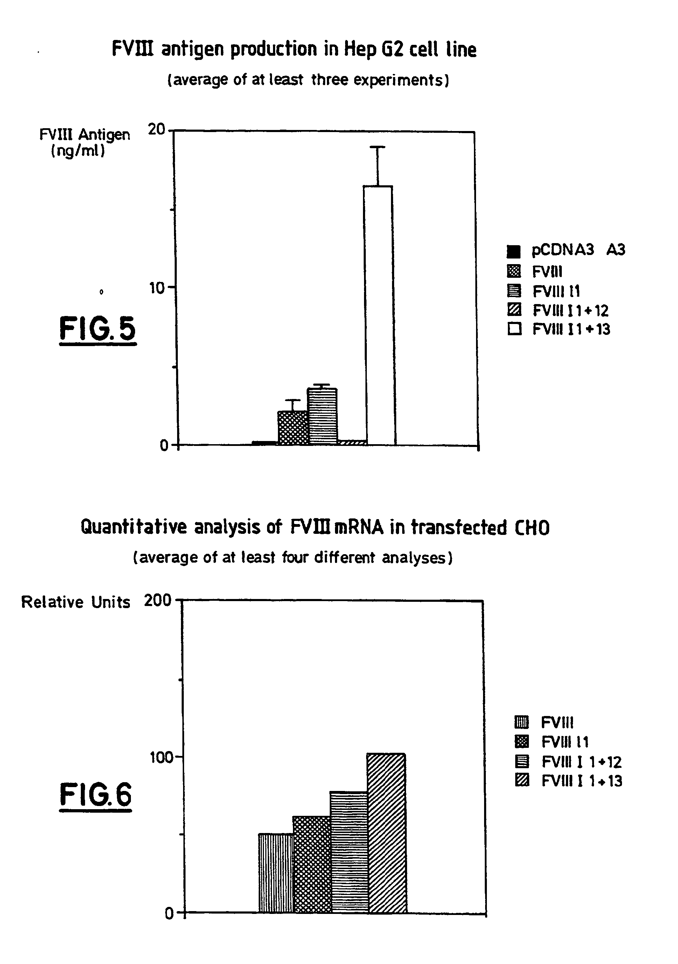

To confirm the data obtained in CHO cells, a second cellular model was chosen. The constructs exhibiting an improvement in the FVIII expression were tested (e.g. FVIII; FVIII I1, FVIII I1+12; FVIII I1+13) using transiently transfected hepatic cell line HepG2. 2 g of circular DNA were added to 12 I of Fugene 6 transfection reagent (Boehringer Mannheim, Meylan, France). After 15 minutes, the complex was added dropwise on a 90 mm dish seeded with 5x10⁶ cells the day before. The transfection mix was incubated for 6h before being replaced by 4 ml of fresh medium. Culture medium was collected 72h later and submitted to coagulation and ELISA assays. As shown in Figure 5, the results obtained using HepG2 cells confirmed the data obtained in CHO cells with the exception of FVIII I1+12 construct which here did not product any detectable FVIII antigen. These data reinforced the potential interest in the FVIII I1+13 construct.

### 5. Analysis of the expression levels

Total RNA of each transfected pool A was extracted with the Rneasy mini Kit (Quiagen), 6.10⁶ cells being used for each extraction. 10 g of total RNA were migrated at 120V, 4 C in a 0.8% agarose gel in phosphate buffer, transferred overnight on Hybond-N Nylon membrane (Amersham) and baked for 2h at 80 C. A FVIII RNA probe, containing the 1.03 kb Sal I-Bgl I fragment, was generated by T7 polymerase using the RNA DIG labelling kit (Boehringer). The membrane was blocked 30 min by the Dig easy Hyb solution (Boehringer) and incubated in the same fresh solution overnight with 500 ng of labeled antisense probe. Washes were conducted as recommended by the manufacturer and the blot was revealed using the DIG detection Kit (Boehringer) analysed and quantified. FVIII signals were compared to ribosomal BET-labeled RNA and to GAPDH signal.

FVIII, FVIII I1 and FVIII I+12 mRNA amounts were very close to each other. FVIII I1+13 mRNA was expressed in larger amounts. These results indicate a correlation between the amount of mRNA and the protein produced for the constructs (see Figure 6).

A two time increase in FVIII I1+13 mRNA lead to a 8 to 9 times protein increase. Therefore, the addition of truncated FIX intron I might play a double role in stabilizing FVIII mRNA but also probably in acting during translation.

The mRNA from FVIII is 4.4 kb long and the differences of 0.3 kb due to a possible non splicing of the TFIXI1 may not be visible in Northern blot. A RT-PCR set of experiments was done on total RNA extracted from the different cell lines. In each case a band corresponding to a spliced mRNA was obtained indicating the splicing of the TFIXI1 from FVIII mRNA.

### 6. Protein characterization

A sheep anti-FVIII antibody from Cedarlane (Hornby, Canada) was purchased and positively tested in a control immunoblot using recombinant FVIII. This antibody was used in immunoblot on cell supernatant but no signal was obtained due to the low amount of secreted antigen. An immunoprecipitation was done on cell supernatant but here again no signal was obtained indicating the inability of this antibody to immunoprecipitate FVIII. An immunoblot was done o Triton-X100 soluble cell lysates. 90mm dishes was lysed with 300 I ice-cold lysis buffer (Hepes 20mM pH7.5, KC1 100mM, MgCl₂ 2mM, Triton X-100 0.5%). Cells were scrapped and centrifuged at 4 C, 10 min at 14000g. Protein concentration was measured with the Dc-protein Assay kit (BioRad, Hercules, USA). 175 g of each cell lysates were loaded on 7.5% acrylamide gel and treated following the Laemmli protocol. After semy-dry transfer (35 min at 400mA), the nitrocellulose membrane was incubated overnight in TBS-T (20mM Tris pH 7.5, NaC1 0.15 M, Tween-20 0.5%). The membrane was then incubated 1h with the anti-FVIII antibody (5 g/ml) in TBS-T. After three washes of 10 min each in TBS-T, the membrane was incubated for 30 min with a rabbit anti-sheep peroxidase coupled antibody (dilution 10⁻⁴ in TBS-T). Extensive washes were conducted before revelation with the ECL system (Amersham).

Among all the lysates, only the cells transfected with the FVIII I1+13 construct gave a positive signal with a correct approximative molecular weight. The FVIII I1+13 appeared as a single band product migrating at a molecular weight corresponding to the Factor VIII heavy chain devoided of the B-domain.

In order to confirm the differences observed in intracellular FVIII amounts, an ELISA was done on Triton-X 100 soluble lysates. The presence of Triton X-100 was shown not to influence the FVIII ELISA test. The value of FVIII antigen present inside the cells confirms the data obtained in Western blot.

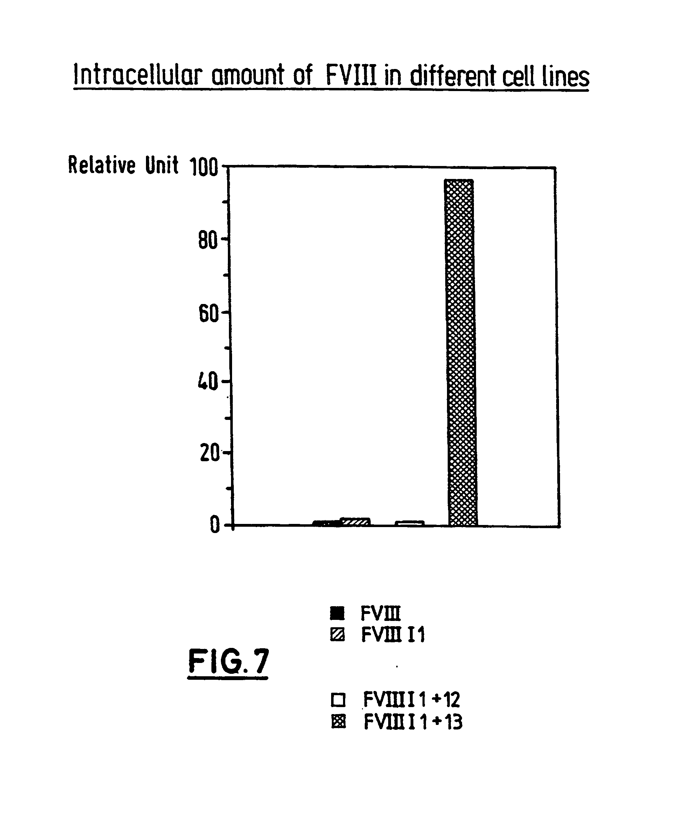

FVIII I1+13 led to a 100 times higher synthesis of the antigen than all other constructs (29 ng/ml vs. 0.3 ng/ml for FVIII) (see Figure 7).

Subject of the invention are, therefore, FVIII-B-domain deleted constructs containing a slightyl modified Factor IX truncated intron I in different locations of the cDNA. Among these constructs a cDNA bearing the truncated intron t in both the FVIII Intron 1 and Intron ¹³ locations led to a 100 times higher intracellular production than all other constructs and a 9 times higher secretion of the protein. This improved production and secretion was observed in two different cell lines: CHO and HepG2 cells. The FVIII produced was fully active on a one stage clotting assay and appears homogenous on immunoblot. The mRNA amount of all the constructs tested differs no more than three times indicating that the benefit observed in the production is coming both from a transcriptional and a translational effect.

The present invention indicates that the production of FVIII may be improved by adding introns in the FVIII cDNA. The advantages of such modified FVIII cDNAs for in vitro FVIII production as well as for human gene therapy by inserting such a cDNA in a suitable transfervector are important for the future treatment of hemophilia A.

### Bibliography

Dorner, A.J., Bole, D.G., and Kaufman, R.J. (1987): The relationship of N-Linked Glycosylation and Heavy Chain-binding Protein Association with the Secretion of Glycoproteins. *J. Cell, Biol.* **105**, 2665-2674.
Fallaux, F.J., Hoeben, R.C., Cramer, S.J., van den Wollenberg, D.J., Briet, E., van Ormondt, H., and van der Eb, A.J. (1996): The human clotting factor VIII cDNA contains an autonomously replicating sequence consensus- and matrix attachment region-like sequence that binds a nuclear factor, represses heterologuos gene expression, and mediates the transcriptional effects of sodium butyrate. *Mol Cell Biol* **16**, 4264-72.
Foster, P.A., and T.S.,, Z. (1989): Factor VIII structure and Function. *Blood Reviews* **3**, 180-191.
Hoeben, R.C., Fallaux, F.J., Cramer, S.J., van den Wollenberg, D.J., van Ormondt, HI, Briet, E., and van der Eb, A.J. (1995): Expression of the blood-clotting factor-VIII cDNA is repressed by a transcriptional silencer located in its coding region. *Blood* **85**, 2447-54.
Kaufman, R.J. (1992): Biological regulation of factor VIII activity. *Annu Rev Med* **43**, 325-39.
Kaufman, R.J., Wasley, L.C., Davies, M.V., Wise, R.J., Israel, D.I., and Dorner, A.j. (1989): Effects of von Willebrand Factor Coexpression on the Synthesis and Secretion of Factor VIII in Chinese Hamster Ovary Cells. *Mol. Cell. Blol.* **9**, 1233-1242.
Koeberl, D.D., Halbert, C.L., Krumm, A., and Miller, A.D. (1995): Sequences within the coding regions of clotting factor VIII and CFTR block transcriptional elongation. *Hum Gene Ther* **6**, 469-79.
Kozak, M. (1997: Recognition of AUG and alternative initiator codons is augmented by G in position +4 but is not generally affected by the nucleotides in positions +5 and +6. *EMBO J.* **16**, 2482-92.
Kurachi S., Hitomi Y., Furukawa M., and Kurachi K. (1995). Role of the Intron 1 in the expression of the human Factor IX gene. J. Biol. Chem. 270, 5276-5281.
Lind, P., Larsson, KI, Spira, J., Spira, J., Sysow Backman, M., Almstedt, A., Gray, E., and Sandberg, H. (1995): Novel forms of B-domain-deleted recombinant factor VIII molecules. Construction and biochemical characterization. *Eur J Biochem* **232**, 19-27.
Lynch, C.M., Israel, D.I., Kaufman, R.J., and Miller, A.D. (1993): Sequences in the coding region of clotting factor VIII act as dominant inhibitors of RNA accumulaton and protein production. *Hum Gene Ther* **4**, 259-72.
Nichols, W.C., Seligsohn, U., Zivellin, A., Terry, V.H., Hertel, C.E., Wheatley, M.A., Moussali, M.J., Hauri, H.-P., Ciavarella, NI, Kaufman, R.J., and Ginsburg, D. (1998): Mutations in the ER-Golgi Intermediate Compartment Protein ERGIC-53 Cause Combined Deficiency of Coagulation Factors V and VIII. *Cell* **93**, 61-70.
Pipe, S.W., Morris, J.A., Shah, JI, and; Kaufman, R.J. (1998): Differential Interaction of Coagulation Factor VIII and Factor V with Protein Chaperones Calnexin and Calreticulin. *J. Biol. Chem*. **273**, 8567-8544.
Pittman D.D., Alerman, E.M., Tomkinson, K.N., Wang, J.H., Giles, A.R., and Kaufman, R.J. (1993: Biochemical, immunological, and in vivo functional characterizaton of B-domain-deleted factor VIII. *Blood* **81**, 2925-35.
Pittman, D.D., Marquette, K.A., and Kaufman, R.J. (1994): Role of the B-domain for factor VIII and factor V expression and function. *Blood* **84**, 4214-25.
Vlot, A.J., Koppelman, S.J., Bouma, B.N., and Sixma, J.J. (1998): Factor VIII and von Willebrand Factor. *Thromb. Haemo.* **79**, 456-465.

### Annex 1

### SEQUENCE LISTING

<110> Aventis Behring GmbH
<120> Modified Factor VIII cDNA
<130> 1999/Z003-Ma1201-C35
<160> 9
<170> Patentln Ver. 2.1
<210> 1
   <211> 30
   <212> DNA
   <213> Homo sapiens
<400> 1
   acacccatgg aaatagagct ctccacctgc 30
<210> 2
   <211> 24
   <212> DNA
   <213> Homo sapiens
<400> 2
   agtcctgaag ctagatctct ctcc 24
<210> 3
   <211> 26
   <212> DNA
   <213> Homo sapiens
<400> 3
   aatatggaga gagatctagc ttcagg 26
<210> 4
   <211> 36
   <212> DNA
   <213> Homo sapiens
<400> 4
   ttctcgtcga cgtcttcttg gttcaatggc attgtt 36
<210> 5
   <211> 34
   <212> DNA
   <213> Homo sapiens
<400> 5
   aagacgtcga cgagaaataa ctcgtactac tctt 34
<210> 6
   <211> 26
   <212> DNA
   <213> Homo sapiens
<400> 6
   agcatgtaga tgctcgccaa taaggc 26
<210> 7
   <211> 28
   <212> DNA
   <213> Homo sapiens
<400> 7
   atttggcggg tggaatgcct tattggcg 28
<210> 8
   <211> 33
   <212> DNA
   <213> Homo sapiens
<400> 8
   acacctcgag tcagtagagg tcctgtgcct cgc 33
<210> 9
   <211> 312
   <212> DNA
   <213> Homo sapiens
<400> 9

gtttgtttat gcatCCTTTT TTAAAATACA TTGAGTATGC TTGCCTTTTA
GATATAGAAA 60
TATCTGATGC TGTCTTCTTC ACTAAATTTT GATTACATGA TTTGACAGCA
ATATTGAAGA 120
GTCTAACAGC CAGCACGCAG GTTGGTAAGT ACTGTGGGAA
CATCACAGAT TTTGGCTCCA 180
TGCCCTAAAG AGAAATTGGC TTTCAGATTA TTTGGATTAA AAACAAAGAC
TTTCTTAAGA 240
GATGTAAAAT TTTCATGATG TTTTCTTTTT TGCTAAAACT AAAGAATTAa
cgcgtattct 300
tttacatttc ag 312

### Annex 2

### Oligonucleotides used for introducing TFIXI1 in FVIII sequence

The oligonucleotide sense is always presented the first one.

**Oligonucleotides used for introducing two restriction sites in TFIXI1:**

FVIII IB-S: 5'-C AT GCA T CC TTT TTT AAA ATA CAT TGA G
Nsi I site

FVIIIB-AS : 5'-A AC GCG T TA ATT CTT TAG TTT TAG CA
Mlu I site

**Oligonucleotides used for the generaton of FVIII compatible ends to clone in FVIII intron 1 location**

### -Generation of I1A

FVIII ATG: 5'-ACA CCC ATG GAA ATA GAG CTC TCC ACC TGC

FVIII IA-AS: 5'-A AT GCA T (AA ACA AAC) CTT GCG TCC ACA GGC
AGC TC Nsi I site FIX splice donor

### -Generation of I1B

FVIII IC-S: 5'-A AC GCG T (AT TCT TTT ACA TTT CAG) ATT TCC TCC
TAG AGT GCC Mlu I site FIX splice donnor

AAA ATCT

FVIII 585-AS: 5'-TTC TCT ACA T AC TAG T AG GGC
endogenous FVIII SpeI site

Oligonucleotides used for the generation of FVIII compatible ends to clone in FVIII intron 12 location

### -Generation of I12A

5' Bgl II : 5'-AAT ATG GAG AGA GAT CTA GCT TCA GG

FVIII 12-AS: 5'-A AT GCA T (AA ACA AAC) TGT GCA TGA TGT TGG
AGG CT Nsi I site FIX splice donor

### -Generation of I12C

FVIII12C-S:5'-A AC GCG T (AT TCT TTT ACA TTT CAG) GCA TCA ATG
GCT ATG TTT    Mlu I site  FIX splice acceptor sequence

4R AS : 5'-TTC TCG TCG ACG TCT TCT TGG TTC AAT GGC ATT GTT

### Oligonucleotides used for the generation of FVIII compatible ends to clone in FVIII intron 13 location

### -Generation of 113A

5' Bgl II : 5'-AAT ATG GAG AGA GAT CTA GCT TCA GG

FVIII 13A-AS: 5'-A AT GCA T (AA ACA AAC) CTG GGT TTT CCA TCG
ACA TGA A Nsi I site FIX splice donor

### -Generation of I13C

FVIII13C-S:5'-A AC GCG T (AT TCT TTT ACA TTT CAG) GTC TAT GGA
TTC TGG GGT Mlu I site FIX splice acceptor

4R AS :5'-TTC TCG TCG ACG TCT TCT TGG TTC AAT GGC ATT GTT

## Claims

1. Modified Factor VIII cDNA, **characterised in that** the B-domain of the wild type Factor VIII cDNA has been deleted and a truncated Factor IX intron 1, SEQ ID NO : 9, has been inserted in one or more different locations normally occupied by introns of the Factor VIII cDNA.

2. Modified Factor VIII cDNA as claimed in claim 1, **characterised in that** the B-domain of the wild-type Factor VIII cDNA is replaced by four arginines.

3. Modified Factor VIII cDNA as claimed in claims 1 and 2, **characterised in that** the truncated Factor IX intron 1), SEQ ID NO : 9, has been inserted at the location normally occupied by introns 1 and/or intron 12 and/or intron 13.

4. Process for the production of Factor VIII, **characterised in that** the production is performed in a cell-line containing a modified Factor VIII cDNA as claims in claims 1 to 3.

5. Transfervector for use in the human gene therapy, **characterised in that** it comprises the modified Factor VIII cDNA of claims 1 to 3.

## Patentansprüche

1. Modifizierte Faktor-VIII-cDNA, **dadurch gekennzeichnet, dass** die B-Domäne der Wildtyp-Faktor-VIII-cDNA entfernt und ein verkürztes Faktor-IX-Intron 1, SEQ ID NO: 9, an einer oder mehreren verschiedenen Positionen eingefügt wurde, die normalerweise durch Introns der Faktor-VIII-cDNA besetzt sind.

2. Modifizierte Faktor-VIII-cDNA nach Anspruch 1, **dadurch gekennzeichnet, dass** die B-Domäne der Wildtyp-Faktor-VIII-cDNA durch vier Argininreste ersetzt ist.

3. Modifizierte Faktor-VIII-cDNA nach den Ansprüchen 1 und 2, **dadurch gekennzeichnet, dass** das verkürzte Faktor-IX-Intron 1, SEQ ID NO: 9, an der Position eingefügt wurde, die normalerweise durch Introns 1 und/oder Intron 12 und/oder Intron 13 besetzt ist.

4. Verfahren zum Herstellen von Faktor VIII, **dadurch gekennzeichnet, dass** das Herstellen in einer Zelllinie erfolgt, die eine modifizierte Faktor-VIII-cDNA nach den Ansprüchen 1 bis 3 enthält.

5. Transfervektor zur Verwendung in der Gentherapie beim Menschen, **dadurch gekennzeichnet, dass** er die modifizierte Faktor-VIII-cDNA nach den Ansprüchen 1 bis 3 enthält.

## Revendications

1. ADNc du facteur VIII modifié, **caractérisé en ce que** le domaine B de l'ADNc du facteur VIII sauvage a été délété et un intron 1 du facteur IX tronqué de séquence SEQ ID NO :9 a été inséré à une ou plusieurs positions différentes normalement occupées par des introns de l'ADNc du facteur VIII.

2. ADNc du facteur VIII modifié tel que revendiqué dans la revendication 1, **caractérisé en ce que** le domaine B de l'ADNc du facteur VIII sauvage est remplacé par quatre arginines.

3. ADNc du facteur VIII modifié tel que revendiqué dans les revendications 1 et 2, **caractérisé en ce que** l'intron 1 du facteur IX tronqué de séquence SEQ ID NO :9 a été inséré à la position normalement occupée par les introns 1 et/ou intron 12 et/ou intron 13.

4. Procédé pour la production du facteur VIII, **caractérisé en ce que** la production est réalisée dans une lignée cellulaire contenant un ADNc du facteur VIII modifié tel que revendiqué dans les revendications 1 à 3.

5. Vecteur de transfert pour l'utilisation en thérapie génique humaine, **caractérisé en ce qu'**il comprend l'ADNc du facteur VIII modifié des revendications 1 à 3.
